# EUROPEAN PATENT SPECIFICATION

(11) **EP 3 800 243 B1**
(45) Date of publication and mention of the grant of the patent: **27.09.2023**
(21) Application number: 19910482.9
(22) Date of filing: 04.06.2019
(51) Int. Cl.: C12N 1/20, C12N 1/02, C02F 3/34

(54) **BACTERIUM DEGRADING LAS AND N AND USE THEREOF**
LAS- UND N-ABBAUENDES BAKTERIUM UND SEINE VERWENDUNG
BACTÉRIES DÉGRADANT LES LAS ET N ET UTILISATION DE CELLES-CI

(30) Priority: 15.01.2019 CN 201910036228
(43) Date of publication of application: 07.04.2021
(73) Proprietor: Agro-Environmental Protection Institute, Ministry of Agriculture and Rural Affairs, Tianjin 300191 (CN); Tianjin Agricultural University, Tianjin 300384 (CN)
(72) Inventor: CHEN, Peizhen, Tianjin 300191 (CN); ZHENG, Xiangqun, Tianjin 300191 (CN); LI, Shiaopeng, Tianjin 300384 (CN)
(74) Representative: Nederlandsch Octrooibureau
(86) International application number: PCT/CN2019/089949
(87) International publication number: WO 2020/147248

(56) References cited:
- CN-A- 1 216 062
- CN-A- 102 899 278
- CN-A- 109 825 449
- CN-A- 110 079 486
- AJU K ASOK ET AL: "Biodegradation of the Anionic Surfactant Linear Alkylbenzene Sulfonate (LAS) by Autochthonous Pseudomonas sp.", WATER, AIR, AND SOIL POLLUTION, KLUWER ACADEMIC PUBLISHERS, DO, vol. 223, no. 8, 19 July 2012 (2012-07-19) , pages 5039-5048, XP035113290, ISSN: 1573-2932, DOI: 10.1007/S11270-012-1256-8
- YU CAI-HONG ET AL: "Isolation and Identification of Ammonia Nitrogen Degradation Strains from Industrial Wastewater", ENGINEERING (ENG ), vol. 04, no. 11, 1 January 2012 (2012-01-01), pages 790-793, XP55901628, ISSN: 1947-3931, DOI: 10.4236/eng.2012.411101 Retrieved from the Internet: URL:https://www.scirp.org/pdf/eng201211000 11_45176216.pdf>
- TANG Meizhen ,LI Tingting,WANG Yanna,WANG Dong,SUN Shishun,HU Pa;npan,LIANG Xiaoqing: "Identification and Characterization of an Efficient Psychrotrophic Bacterial Strain Isolated from Artificial Wetlands", Acta Scientiae Circumstantiae, vol. 33, no. 3, 6 March 2016 (2016-03-06), page 708, XP055763804, ISSN: 0253-2468, DOI: 10.13671/j.hjkxxb.2013.03.004

## Description

### FIELD OF THE INVENTION

The present invention belongs to the technical field of bioremediation, and particularly relates to a LAS-degrading and N-removing bacterium and applications thereof.

### BACKGROUND OF THE INVENTION

With the rapid improvement of the living conditions of rural residents in China, the discharge of rural domestic sewage has also been increasing year by year, and nitrogen (N) is one of the main pollutants in domestic sewage. Studies have shown that among the main pollutants which cause eutrophication of Lake Taihu, 25% of nitrogen and 60% of phosphorus are derived from rural domestic sewage. Although nitrogen is one of the nutrients largely required by plant growth, a significant increase in nitrogen in water would lead to a range of environmental problems, such as consumption of dissolved oxygen in water, production of cancerogenic nitrosamines, and eutrophication of water, posing a huge threat to the ecological environment and human health. Therefore, removal of nitrogen from polluted water is of great significance for protecting the environment, developing the economy and ensuring health. Linear alkylbenzene sulfonates (LAS) are a class of anionic surfactants widely used as household detergents and cleaning agents. According to statistics, in China detergents increased from about 1 million tons in 1985 to about 4.6 million tons in 2005. A large amount of LAS discharged into waterbody would not only result in production of foam that affects the natural landscape of water, but also have a significant influence on the redox capacity and enzymatic activity of microorganisms, which reduces the activity of the microorganisms and causes them to lose some of their original functions in the environment. Until now LAS have become another important organic pollutant in domestic sewage, and it is highly important to remove LAS and nitrogen from rural domestic sewage. Therefore, there is still a demand for bacteria that can be used for rural sewage treatment.

### SUMMARY OF THE INVENTION

It is an object of the present invention to provide a bacterium which degrades LAS (linear alkylbenzene sulfonates) and removes N (nitrogen). The bacterium was isolated and screened from the sewage of the rural septic tank in Ankang, Shaanxi (China), and can be used for sewage treatment.

The LAS-degrading and N-removing bacterium provided by the present invention is taxonomically classified as *Pseudomonas* sp., and was deposited in China General Microbiological Culture Collection Center (CGMCC; address: No. 3, Yard No. 1, West Beichen Road, Chaoyang District, Beijing, China) on September 18^{th}, 2018, with the accession number of CGMCC 16502.

Preferably, the 16S rDNA sequence of the strain of the bacterium is represented by SEQ ID NO: 1, having a length of 1498 bp.

Preferably, the strain of the bacterium has the following phenotypic characteristics: when cultured at 36°C for 24 h, the bacterial colonies are light yellow, circular, and opaque, and have a wet surface and a smooth margin; the bacteria cells are rod-shaped, 0.4~0.8 µm × 1.8~4.7 µm in size, arranged individually or in pairs, and gram-negative.

Preferably, the strain of the bacterium is characterized by a (G+C)mol% of 63.4%.

Preferably, the strain of the bacterium has the following characteristics: the quinone components are ubiquinone Q8 and ubiquinone Q9, and their relative contents are 57.24% and 42.76%, respectively.

Preferably, the strain of the bacterium has the following characteristics: the major fatty acid components (relative content > 10%) are: C_{16:0}, Summed Feature 3 (C_{16:1}ω7c or C₁₆:₁ω6c), and Summed Feature 8 (C₁₈:₁ω7c or C₁₈: ₁ω6c), and their relative contents are 31.16%, 14.85%, and 12.32%, respectively.

Preferably, the strain of the bacterium has the following characteristics: the cell wall-characteristic DAP component is *meso-*DPA; and the cell wall-characteristic sugar component is ribose.

Preferably, the strain of the bacterium has the following characteristics: the bacterial polar lipids comprise diphosphatidylglycerol, phosphatidylglycerol, phosphatidylethanolamine, phosphatidylcholine, an unidentified phospholipid, an unidentified lipid, and an unidentified aminophospholipid.

Preferably, the hybridization homology of the bacterial strain with the model strain *Pseudomonas guangdongensis* CCTCC AB2012022^{T} is 35.4%.

Preferably, the bacterium shows its LAS-degrading effect over an initial LAS concentration of 0-400 mg/L; and the bacterium shows its ammoniacal nitrogen-removing effect over an initial ammoniacal nitrogen concentration of 0-200 mg/L.

More preferably, the bacterium shows its LAS-degrading effect over an initial LAS concentration of 0-200 mg/L; and the bacterium shows its ammoniacal nitrogen-removing effect over an initial ammoniacal nitrogen concentration of 0-100 mg/L.

Even more preferably, the bacterium can simultaneously degrade LAS and remove N.

According to the morphological characteristics and the physiological and biochemical characteristics of the bacterium and the results of aligning its 16S rDNA to Genbank, a phylogenetic tree was constructed and the strain was comprehensively identified as *Pseudomonas* sp.

In particular, the sewage is rural domestic sewage.

In addition, the present invention provides a use of the bacterium for decontamination of rural domestic sewage, wherein the rural domestic sewage has a LAS concentration of 0 to 400 mg/L and/or an ammoniacal nitrogen concentration of 0 to 200 mg/L.

The LAS-degrading and N-removing bacterium according to the present invention has the following beneficial effects as compared with the prior art.

Under aerobic conditions, the bacterium of the present invention can assimilate LAS as the sole carbon source and ammoniacal nitrogen as the sole nitrogen source. By this characteristic of the strain, it can be directly applied to the rural domestic sewage containing LAS, such as the kitchen, bath sewage, etc., and the removal of N from the sewage with a high LAS content can be realized. As a result, it would have good application prospects and social benefits.

The LAS-degrading and N-removing bacterium according to the present invention is an indigenous microorganism screened from rural domestic sewage with a high salt content, and their application to the rural domestic sewage with a high salt content will achieve a considerable economic benefit, greatly save the cost for sewage treatment, and have no safety concern about the strain, thereby being suitable for use in rural areas of China where the gathering places are scattered. The bacterial strain according to the present invention has an excellent effect of degrading LAS-N composite pollutants, and addition of the bacteria can effectively improve the efficiency of the indigenous microorganism in removing N and LAS from rural domestic sewage.

It can be understood that, in the present application, "LAS-degrading and N-removing bacterium", "bacterium which degrades LAS and removes N", *"Pseudomonas"* and *"Pseudomonas* sp." have the same meaning, and "bacterium", "species" and "strain" have the same meaning in some places. LAS refers to linear alkylbenzene sulfonate. Both N and "nitrogen" refer to the nitrogen element.

### DEPOSITION INFORMATION

Bacterium name: LAS-degrading and N-removing bacterium
Taxonomy: *Pseudomonas* sp.
Strain No.: A-1
Depository: China General Microbiological Culture Collection Center
Depository abbreviation: CGMCC
Address of Depository: No. 3, Yard No. 1, West Beichen Road, Chaoyang District, Beijing, China
Date of deposit: September 18^{th}, 2018
Depository Accession Number: CGMCC 16502

### BRIEF DESCRPTION OF THE DRAWINGS

Figure 1 is an electron micrograph showing the morphology of cells of the LAS-degrading and N-removing bacterium according to the present invention.
Figure 2 is a photograph showing the morphology of colonies of the LAS-degrading and N-removing bacterium according to the present invention.
Figure 3 is a 16S rDNA sequence genetic tree of the LAS-degrading and N-removing bacterium according to the present invention and related species.
Figure 4 is a diagram showing the nitrogen removing effect of the LAS-degrading and N-removing bacterium according to the present invention at different initial concentrations of ammoniacal nitrogen.

### DETAILED DESCRPTION OF THE INVENTION

### Example 1 Isolation and identification of the bacterial strain of the present invention

### (1) Medium

① Medium for nitrogen removal: 0.48 g of ammonium sulfate, 4.71 g of succinic acid and a 50 mL trace element solution were dissolved and then made to a volume of 1 L with distilled water, with the pH controlled between 7.0 and 7.2. (for a solid medium, 2% agar was added). The trace element solution: 6.5 g of K₂HPO₄·3H₂O, 2.5 g of MgSO₄·7H₂O, 2.5 g of NaCl, 0.05 g of FeSO₄·7H₂O, and 0.04 g of MnSO₄·H₂O were dissolved and made to a volume of 1 L with distilled water.
② LAS-degrading selective medium: 0.48 g of ammonium sulfate, 4.71 g of succinic acid, 0.10 g of LAS, and a 50 mL trace element solution. The trace element solution: 6.5 g of K₂HPO₄·3H₂O, 2.5 g of MgSO₄·7H₂O, 2.5 g of NaCl, 0.05 g of FeSO₄·7H₂O, and 0.04 g of MnSO₄·H₂O, which were dissolved and made to a volume of 1 L with distilled water.

### (2) Isolation and screening of nitrogen removing strains

10 mL of sewage from the septic tank in Ankang, Shaanxi was taken with a sterile pipette, placed in a 250 ml Erlenmeyer flask (containing 100 mL sterile medium for nitrogen removal), and cultured at 30°C on a shaker at 160 rpm. Each passage was cultured for three days. The presence of nitrobacteria was detected by using a diphenylamine reagent. The bacterial liquid was cultured until the presence of nitrobacteria was detected in the medium.

The culture liquid containing the nitrobacteria was plated by limiting dilution, and cultured in an incubator at 37°C for 48 hours. Afterwards single colonies were picked, and inoculated by the streak plate method for multiple times. When no contaminating bacteria were observed under a microscope, the purification of the strain was completed.

The purified strain was subjected to a screening experiment for ammoniacal nitrogen removal. The strains were individually inoculated in the medium for nitrogen removal, and cultured in an incubator at 37°C for 48 hours. The ammoniacal nitrogen removal was determined, and the strains with the ammoniacal nitrogen removal higher than 85% were selected as the nitrogen-removing strains.

### (3) Screening for nitrogen-removing and LAS-degrading strains

The selected nitrogen-removing strains were inoculated into the liquid LAS-degrading selective medium in an inoculation amount of 10%, and cultured on a shaker for 48 hours, and the degradation of LAS was measured. A strain showing a degradation higher than 80% is a LAS-degrading and N-removing bacterium.

### (4) A LAS-degrading and

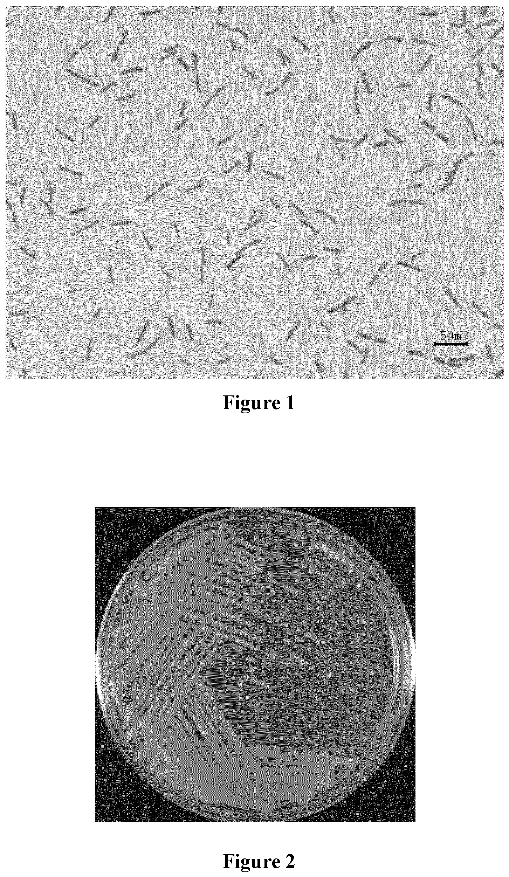

N-removing bacterium was identified by screening. Figure 1 is an electron micrograph showing the morphology of cells of the LAS-degrading and N-removing bacterium according to the present invention. Figure 2 is a photograph showing the morphology of colonies of the LAS-degrading and N-removing bacterium according to the present invention. It can be seen from Figure 1 and Figure 2 that the strain of bacterium has the following phenotypic characteristics: when cultured at 36°C for 24h, the bacterial colonies are light yellow, circular, and opaque, and have a wet surface and a smooth margin; the bacteria cells are rod-shaped, 0.4~0.8 µm × 1.8~4.7 µm in size, arranged individually or in pairs, and gram-negative.

Furthermore, the bacterial strain was assayed and has the following characteristics: (G+C)mol% is 63.4%; the quinone components are ubiquinone Q8 and ubiquinone Q9, and their relative contents are 57.24% and 42.76%, respectively; the major fatty acid components (relative content > 10%) are: C_{16:0}, Summed Feature 3 (C_{16:1}ω7c or C_{16:1}ω6c), and Summed Feature 8 (C₁₈:₁ω7c or C₁₈: ₁ω6c), and their relative contents are 31.16%, 14.85% and 12.32%, respectively; the cell wall-characteristic DAP component is *meso-*DPA; and the cell wall-characteristic sugar component is ribose; the polar lipid of this strain comprises diphosphatidylglycerol, phosphatidylglycerol, phosphatidylethanolamine, phosphatidylcholine, an unidentified phospholipid, an unidentified lipid, and an unidentified aminophospholipid; and the hybridization homology of the bacterial strain with the model strain *Pseudomonas guangdongensis* CCTCC AB2012022^{T} is 35.4%.

### (5)16S rDNA sequence analysis and phylogenetic analysis

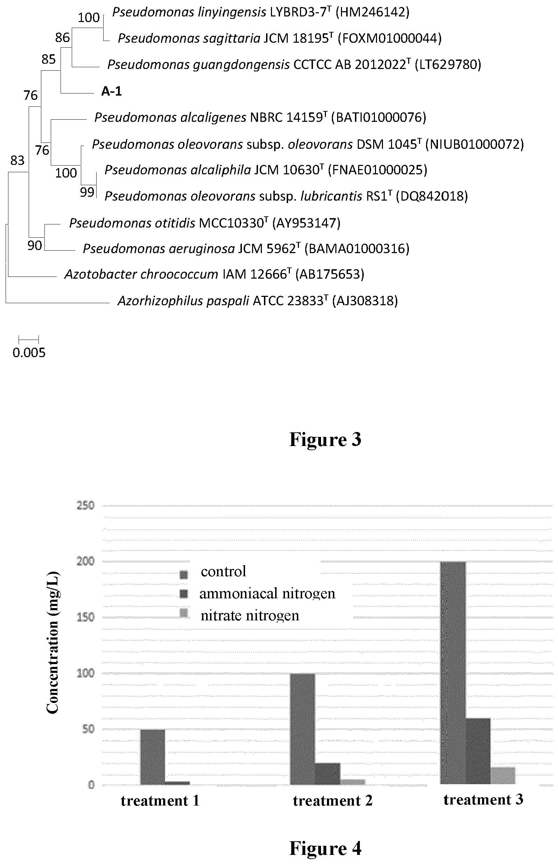

The 16S rDNA gene sequence was 1498 bp in length as confirmed by PCR amplification and sequencing of the 16S rDNA (see SEQ ID NO: 1). Through the alignment by submission to Genbank, it was found that the LAS-degrading and N-removing bacterium according to the present invention can have a homology as high as 97.88% with *Pseudomonas linyingensis* LYBRD3-7^{T}. The 16S rDNA sequence genetic tree of the strain and some related species was generated with the software MEGA 5.0 by the neighbor-joining method, and the similarity was repeatedly calculated for 1,000 times. In Figure 3, the genetic tree nodes only showed those with Bootstrap values greater than 50% (see Figure 3; Figure 3 is a 16S rDNA sequence genetic tree of the strain of the present invention and related species).

### (6)The physiological and biochemical results of the LAS-degrading and N-removing bacterium according to the present invention are shown in Table 2 below.

**Table 2 Physiological and Biochemical Characteristics:**

| | | | | | | | |
|---|---|---|---|---|---|---|---|
| Ala-Phe-Pro arylamidase | - | adonitol | - | L-pyrrolidonyl arylamidase | - | L-Arabitol | - |
| D-cellobiose | - | β-galactosidase | - | H₂S Production | - | β-N-acetylamino-glucosidase | - |
| Glutamyl arylamidase pNA | - | D-glucose | - | γ-glutamyl-transferase | - | fermentation/glucose | - |
| β-Glucosidase | - | D-maltose | + | D-mannitol | - | D-mannose | - |
| P-xylosidase | - | β-Alanine arylamidase pNA | - | L-proline arylamidase | + | lipase | - |
| palatinose | - | tyrosine arylamidase | + | urease | - | D-sorbitol | - |
| sucrose | - | D-tagatose | - | D-trehalose | - | Citrate (sodium) | - |
| malonate | - | 5-keto-D-Gluconate | - | L-lactate alkalinisation | - | α-glucosidase | - |
| succinate alkalinisation | - | β-N-Acetyl-galactosaminidase | - | α-galactosidase | - | phosphatase | - |
| Glycine arylaminase | | ornithine decarboxylase | | lysine decarboxylase | | L-Histidine assimilation | |
| coumaric acid | - | β-glucuronidase | - | O/129 resistance | + | Glu-Gly-Arg-arylamidase | - |
| L-malate assimilation | + | ELLMAN | - | L-lactate assimilation | - | | |

| | | | | | | | |
|---|---|---|---|---|---|---|---|
| Symbol Description: "+", positive; "-", negative. | | | | | | | |

### Example 2 LAS-degrading test

The activated LAS-degrading and N-removing bacterium of the present invention was respectively inoculated into the media having LAS concentrations of 50 mg/L, 100 mg/L, 200 mg/L and 400 mg/L by a dilution coating method, with an inoculum amount of 1%. One blank control group and 3 parallel test groups were set up. After culturing under shaking for 48 hours, the degradation of LAS was determined. The results are shown in Table 3 below.

**Table 3. Degradation of the strains of the present invention at different initial concentrations of LAS**

| | | | | | |
|---|---|---|---|---|---|
| Initial concentration (mg/L) | 0 | 50 | 100 | 200 | 400 |
| Removal of LAS (%) | - | 87 | 82 | 78 | 50 |

It can be seen from the results in Table 3 that the LAS-degrading and N-removing bacterium of the present invention can degrade LAS, and can exert this LAS-degrading function at an initial LAS concentration from 0 to 400 mg/L. In particular, the degradation of LAS was higher at the initial LAS concentration from 0 to 200 mg/L.

### Example 3 Nitrogen removing Effect Experiment

The LAS-degrading and N-removing bacterial strain of the present invention was inoculated into 100 mL media for nitrogen removal at different nitrogen levels, with initial ammoniacal nitrogen concentrations of 50 mg/L (Treatment 1), 100 mg/L (Treatment 2) and 200 mg/L (Treatment 3), respectively. A medium not inoculated with the strain was used as a blank control. The media were cultured in an incubator at 37°C under shaking at 160 rpm for 30 h. The culture solution was centrifuged at 10,000 rpm for 6 minutes, and filtered through a 0.45 µm filter. The contents of ammoniacal nitrogen and nitrate nitrogen in the culture solution were measured. The detailed measurement results are shown in Figure 4. Figure 4 is a diagram showing the nitrogen removing effect of the LAS-degrading and N-removing bacterial strain of the present invention at different initial ammoniacal nitrogen concentrations.

It can be seen from Figure 4 that the LAS-degrading and N-removing bacterial strain of the present invention showed removals of 92%, 80% and 70% at different initial ammoniacal nitrogen concentrations, respectively, indicating that the LAS-degrading and N-removing bacterium of the present invention had a good nitrogen removing function, and can always exert this nitrogen removing function at an initial ammoniacal nitrogen concentration less than 200 mg/L, where the nitrogen removing effect was even better when the initial ammoniacal nitrogen concentration was less than 100 mg/L.

### Example 4 Degradation Experiment on Rural Domestic Sewage

(1) In the Tanjiawan area of Shiyan, Hubei Province (China), the domestic sewage of the rural septic tank was subjected to grid filtration and sedimentation, and 20 L of the supernatant was added to an integrated sewage treatment apparatus. Test results: the pH of wastewater was 7.2, the ammoniacal nitrogen concentration was 60 mg/L, CODcr was 300 mg/L, and LAS was 5 mg/L.
(2) The liquid containing the LAS-degrading and N-removing bacterium of the present invention was directly added to the integrated sewage treatment apparatus containing the 20 L of rural domestic sewage, in an inoculum amount of 10% by volume. The culturing temperature was 30°C. The pH of the target water did not need to be controlled during the sewage decontamination process, and aeration was performed for 1 hour every day. Results: after 2 days of treatment, the removal of ammoniacal nitrogen from the water can reach 88%, the removal of CODcr can reach 60%, and the degradation of LAS was 97%.

## Claims

1. A LAS-degrading and N-removing bacterium, taxonomically classified as *Pseudomonas* sp., deposited in China General Microbiological Culture Collection Center on September 18^{th}, 2018, with the accession number of CGMCC 16502.

2. The bacterium according to claim 1, **characterized in that** the 16S rDNA sequence of the strain of the bacterium is SEQ ID NO: 1.

3. The bacterium according to claim 1, **characterized in that** the strain of the bacterium has the following phenotypic characteristics:
when cultured at 36°C for 24 h, the bacterial colonies are light yellow, circular and opaque, and have a wet surface and a smooth margin; and
the bacterial cells are rod-shaped, 0.4~0.8 µm × 1.8~4.7 µm in size, arranged individually or in pairs, and gram-negative.

4. The bacterium according to claim 1, **characterized in that** the strain of the bacterium has the following characteristics:
(G+C)mol% is 63.4%; and
the quinone components are ubiquinone Q8 and ubiquinone Q9, and their relative contents are 57.24% and 42.76%, respectively.

5. The bacterium according to claim 1, **characterized in that** the strain of the bacterium has the following characteristics: the major fatty acid components are: C_{16:0}, Summed Feature 3 (C_{16:1}ω7c or C_{16:1}ω6c), and Summed Feature 8 (C_{18:1}ω7c or C_{18:1}ω6c), and their relative contents are 31.16%, 14.85% and 12.32%, respectively.

6. The bacterium according to claim 1, **characterized in that** the strain of the bacterium has the following characteristics: the cell wall-characteristic DAP component is *meso-*DPA; and the cell wall-characteristic sugar component is ribose.

7. The bacterium according to claim 1, **characterized in that** the strain of the bacterium has the following characteristics: the bacterial polar lipids comprise diphosphatidylglycerol, phosphatidylglycerol, phosphatidylethanolamine, phosphatidylcholine, an unidentified phospholipid, an unidentified lipid and an unidentified aminophospholipid.

8. The bacterium according to claim 1, **characterized in that** the bacterium shows its LAS-degrading effect over an initial LAS concentration of 0-400 mg/L; and the bacterium shows its ammoniacal nitrogen-removing effect over an initial ammoniacal nitrogen concentration of 0-200 mg/L;
preferably, the bacterium shows its LAS-degrading effect over an initial LAS concentration of 0-200 mg/L; and the bacterium shows its ammoniacal nitrogen-removing effect over an initial ammoniacal nitrogen concentration of 0-100 mg/L.

9. Use of the bacterium according to any one of claims 1 to 8 for decontamination of rural domestic sewage, wherein the rural domestic sewage has a LAS concentration of 0 to 400 mg/L and an ammoniacal nitrogen concentration of 0 to 200 mg/L.

## Patentansprüche

1. LAS-abbauendes und N-entfernendes Bakterium, taxonomisch klassifiziert als *Pseudomonas* sp., hinterlegt im China General Microbiological Culture Collection Center am 18. September 2018 mit der Zugangsnummer CGMCC 16502.

2. Bakterium nach Anspruch 1, **gekennzeichnet dadurch, dass** die 16S-rDNA-Sequenz des Stammes des Bakteriums SEQ ID NO: 1 ist.

3. Bakterium nach Anspruch 1, **gekennzeichnet dadurch, dass** der Stamm des Bakteriums die folgenden phänotypischen Merkmale aufweist:
wenn die Bakterienkolonien 24 Stunden lang bei 36 °C kultiviert werden, sind sie hellgelb, rund und lichtundurchlässig und haben eine feuchte Oberfläche und einen glatten Rand; und
die Bakterienzellen sind stäbchenförmig, 0,4-0,8 µm × 1,8~4,7 µm groß, einzeln oder paarweise angeordnet und gramnegativ.

4. Bakterium nach Anspruch 1, **gekennzeichnet dadurch, dass** der Stamm des Bakteriums die folgenden Merkmale aufweist:
(G+C)mol% ist 63,4 %; und
die Chinon-Komponenten sind Ubichinon Q8 und Ubichinon Q9 und ihre relativen Gehalte betragen 57,24 % bzw. 42,76 %.

5. Bakterium nach Anspruch 1, **gekennzeichnet dadurch, dass** der Stamm des Bakteriums die folgenden Merkmale aufweist: die Hauptfettsäurekomponenten sind: C_{16:0}, Summenmerkmal 3 (C_{16:1}ω7c oder C_{16:1}ω6c) und Summenmerkmal 8 (C_{18:1}ω7c oder C_{18:1}ω6c) und ihre relativen Gehalte betragen 31,16 %, 14,85 % bzw. 12,32 %.

6. Bakterium nach Anspruch 1, **gekennzeichnet dadurch, dass** der Stamm des Bakteriums die folgenden Merkmale aufweist: die für die Zellwand charakteristische DAP-Komponente ist meso-DPA; und die für die Zellwand charakteristische Zuckerkomponente ist Ribose.

7. Bakterium nach Anspruch 1, **gekennzeichnet dadurch, dass** der Stamm des Bakteriums die folgenden Merkmale aufweist: die bakteriellen polaren Lipide umfassen Diphosphatidylglycerin, Phosphatidylglycerin, Phosphatidylethanolamin, Phosphatidylcholin, ein nicht identifiziertes Phospholipid, ein nicht identifiziertes Lipid und ein nicht identifiziertes Aminophospholipid.

8. Bakterium nach Anspruch 1, **gekennzeichnet dadurch, dass** das Bakterium seine LAS-abbauende Wirkung bei einer anfänglichen LAS-Konzentration von 0-400 mg/L zeigt; und das Bakterium seine Ammoniakstickstoff-entfernende Wirkung bei einer anfänglichen Ammoniakstickstoff-Konzentration von 0-200 mg/L zeigt;
wobei vorzugsweise das Bakterium seine LAS-abbauende Wirkung bei einer anfänglichen LAS-Konzentration von 0-200 mg/L zeigt; und das Bakterium seine Ammoniakstickstoff-entfernende Wirkung bei einer anfänglichen Ammoniakstickstoff-Konzentration von 0-100 mg/L zeigt.

9. Verwendung des Bakteriums nach einem der Ansprüche 1 bis 8 zur Dekontamination ländlicher häuslicher Abwässer, wobei die ländlichen häuslichen Abwässer eine LAS-Konzentration von 0 bis 400 mg/L und eine Ammoniakstickstoff-Konzentration von 0 bis 200 mg/L aufweisen.

## Revendications

1. Bactérie dégradant les LAS et éliminant l'azote, classée taxonomiquement comme *Pseudomonas* sp., déposée au Centre général chinois de collecte de cultures microbiologiques le 18 septembre 2018, sous le numéro d'accès CGMCC 16502.

2. Bactérie selon la revendication 1, **caractérisée en ce que** la séquence d'ADNr 16S de la souche bactérienne est SEQ ID NO : 1.

3. Bactérie selon la revendication 1, **caractérisée en ce que** la souche bactérienne a les caractéristiques phénotypiques suivantes :
lorsqu'elles sont cultivées à 36 °C pendant 24 h, les colonies bactériennes sont jaune clair, circulaires et opaques, et ont une surface humide et une marge lisse ; et
les cellules bactériennes sont en forme de bâtonnet, taille de 0,4~0,8 µm × 1,8-4,7 µm, disposées individuellement ou par paires, et gram-négatives.

4. Bactérie selon la revendication 1, **caractérisée en ce que** la souche bactérienne a les caractéristiques suivantes :
(G+C)mol % est de 63,4 % ; et
les composants de quinone sont ubiquinone Q8 et ubiquinone Q9, et leurs teneurs relatives sont 57,24 % et 42,76 %, respectivement.

5. Bactérie selon la revendication 1, **caractérisée en ce que** la souche bactérienne a les caractéristiques suivantes : les principaux composants d'acides gras sont : C_{16:0}, Caractéristiques Cumulées 3 (C_{16:1}ω7c ou C_{16:1}ω6c), et Caractéristiques Cumulées 8 (C_{18:1}ω7c ou C_{18:1}ω6c), et leurs teneurs relatives sont 31,16 %, 14,85 % et 12,32 %, respectivement.

6. Bactérie selon la revendication 1, **caractérisée en ce que** la souche bactérienne a les caractéristiques suivantes : le composant DAP caractéristique de la paroi cellulaire est *méso-DPA* ; et le composant de sucre caractéristique de la paroi cellulaire est ribose.

7. Bactérie selon la revendication 1, **caractérisée en ce que** la souche bactérienne a les caractéristiques suivantes : les lipides polaires bactériens comprennent diphosphatidylglycérol, phosphatidylglycérol, phosphatidyléthanolamine, phosphatidylcholine, un phospholipide non identifié, un lipide non identifié et un aminophospholipide non identifié.

8. Bactérie selon la revendication 1, **caractérisée en ce que** la bactérie montre son effet dégradant des LAS sur une concentration initiale en LAS de 0 à 400 mg/L ; et la bactérie montre son effet d'élimination de l'azote ammoniacal sur une concentration initiale en azote ammoniacal de 0 à 200 mg/L ;
de préférence, la bactérie montre son effet dégradant des LAS sur une concentration initiale en LAS de 0 à 200 mg/L ; et la bactérie montre son effet d'élimination de l'azote ammoniacal sur une concentration initiale en azote ammoniacal de 0 à 100 mg/L.

9. Utilisation de la bactérie selon l'une quelconque des revendications 1 à 8 pour la décontamination d'eaux usées domestiques rurales, où les eaux usées domestiques rurales ont une concentration en LAS de 0 à 400 mg/L et une concentration en azote ammoniacal de 0 à 200 mg/L.
